Europäisches Patentamt

European Patent Office

Office européen des brevets

(11) Publication number: **0 459 480 A1**

# EUROPEAN PATENT APPLICATION

(21) Application number: 91108858.1

(51) Int. Cl.5: **A61B 5/0265**

(22) Date of filing: **29.05.91**

(30) Priority: **31.05.90 JP 139908/90**

(43) Date of publication of application:
**04.12.91 Bulletin 91/49**

(84) Designated Contracting States:
**DE FR GB NL**

(71) Applicant: **Nihon Kohden Corporation**
**31-4, 1-chome, Nishiochiai**
**Shinjuku-ku Tokyo(JP)**

(72) Inventor: **Mohri, Hiromichi, Nihon Kohden**
**Corporation**
**31-4, Nishiochiai 1-chome, Shinjuku-ku**
**Tokyo(JP)**
Inventor: **Ogawa, Keikistu, Nihon Kohden**
**Corporation**
**31-4, Nishiochiai 1-chome, Shinjuku-ku**

**Tokyo(JP)**
Inventor: **Ose, Sei, Nihon Kohden Corporation**
**31-4, Nishiochiai 1-chome, Shinjuku-ku**
**Tokyo(JP)**
Inventor: **Takeda, Sunao, Nihon Kohden**
**Corporation**
**31-4, Nishiochiai 1-chome, Shinjuku-ku**
**Tokyo(JP)**
Inventor: **Sugimoto, Yuji, Nihon Kohden**
**Corporation**
**31-4, Nishiochiai 1-chome, Shinjuku-ku**
**Tokyo(JP)**

(74) Representative: **Patentanwälte Grünecker,**
**Kinkeldey, Stockmair & Partner**
**Maximilianstrasse 58**
**W-8000 München 22(DE)**

(54) **Electromagnetic blood flowmeter probe.**

(57) An electromagnetic blood flowmeter probe comprises an electrode portion (I) having electrodes (11) provided on a hollow bobbin (10) through which a blood flow passes, an exciter coil portion (II) having a body (15) in which an exciter coil (16) for applying a magnetic field to the blood flow is embedded, and a positioning device (14,19) for fixing a relative position between the electrode portion and the exciter coil portion when they are connected together is provided on the bobbin of the electrode portion and the body of the exciter coil portion.

FIG. 1

## BACKGROUND OF THE INVENTION

This invention relates to a probe of an electromagnetic blood flowmeter for measuring a blood flow, and more particularly to an electromagnetic blood flowmeter probe of the separating type in which an electrode portion incorporated in a blood circuit is disposable.

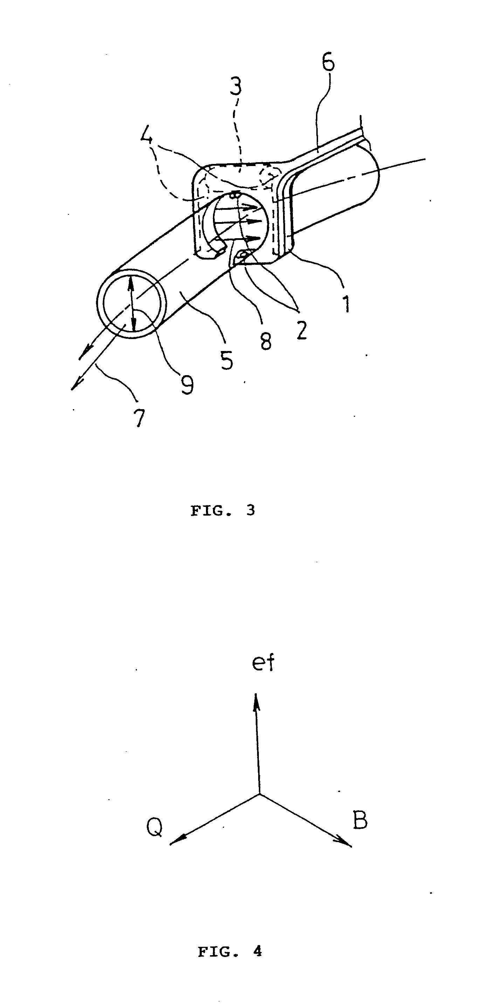

Fig. 3 shows the construction of a conventional electromagnetic blood flowmeter probe. In this probe, an electrode portion and an exciter coil portion are formed integrally with each other, and electrodes 2, an exciter coil 3 and an iron core 4 are embedded in a body 1. The body 1 has at its central portion a hole through which a blood vessel wall 5 is adapted to be passed. The probe is adapted to be connected to a flowmeter (not shown) by a cord 6. A blood flow 7 passes through the interior of the blood vessel wall 5. A magnetic flux 8 is applied to the blood flow 7. Reference numeral 9 denotes the inner diameter of the blood vessel.

When the magnetic field is applied by the exciter coil 3 to the blood flow, an electromotive force proportional to the velocity of this blood flow is produced. This electromotive force is detected by the two electrodes 2 provided respectively on the opposite sides of the blood vessel wall 5, and is inputted to the blood flowmeter via the cord 6. This electromotive force is amplified by this blood flowmeter, and is recorded and displayed as a detection value of the blood flow amount.

The principle of the above-mentioned electromagnetic blood flowmeter can be explained by Faraday's law of electromagnetic induction. More specifically, as shown in Fig. 4, when the blood flows in a direction perpendicular to the above magnetic field, the electromotive force ef is produced in a direction perpendicular to this magnetic field and the blood flow. The magnitude of this electromotive force ef can be expressed by the following formula:

$$ef = BDV \times 10^{-8} \text{ [V]} \qquad (1)$$

B: magnetic flux density [Gauss]
D: distance between the electrodes [cm]
V: mean velocity of the blood flow [cm/s]

Therefore, if the magnetic flux density B and the distance D between the electrodes are made constant, the mean flow velocity V can be found by measuring the above electromotive force ef, and the flow rate Q can also be found by the following formula:

$$Q = \pi D^2 V/4 \qquad (2)$$

However, in the above conventional electromagnetic blood flowmeter probe, the sensitivity varies with the change of the magnetic flux density, and therefore it is necessary to fix the mechanical position of the exciter coil relative to the electrodes, and it is difficult to separate the electrode portion from the exciter coil portion. The electrodes are provided integrally with the exciter coil in order to make the magnetic flux density constant, and therefore when measuring the flow rate in a blood circuit, for example, of a dialysis treatment machine, a ventricular assist device or an oxygenerator, parts of the blood circuit, such as tubes, are disposable when incorporating the probe into the blood circuit; however, there is a problem that the probe body is not disposable since it is expensive. Further, when the probe is to be re-used, it must be sterilized separately from the blood circuit parts, and thus there is another problem that the incorporation is cumbersome.

## SUMMARY OF THE INVENTION

The present invention has been made in view of the above problems, and an object of the invention is to provide an electromagnetic blood flowmeter probe in which an electrode portion is disposable together with blood circuit parts, and the incorporation is not cumbersome.

An electromagnetic blood flowmeter probe of the present invention comprises an electrode portion having electrodes provided on a hollow bobbin through which a blood flow passes; an exciter coil portion having a body in which an exciter coil for applying a magnetic field to said blood flow is embedded; and a positioning mechanism for fixing a relative position between said electrode portion and said exciter coil portion when they are connected together is provided on said bobbin of said electrode portion and said body of said exciter coil portion.

In the electromagnetic blood flowmeter probe of the present invention, the exciter coil portion is detachably connected to the electrode portion having the electrodes, and at this time by the positioning mechanism provided on the hollow bobbin of the electrode portion and the body of the exciter coil portion, the mechanical relative position between the electrode portion and the exciter coil portion is fixed.

## BRIEF DESCRIPTION OF THE DRAWINGS

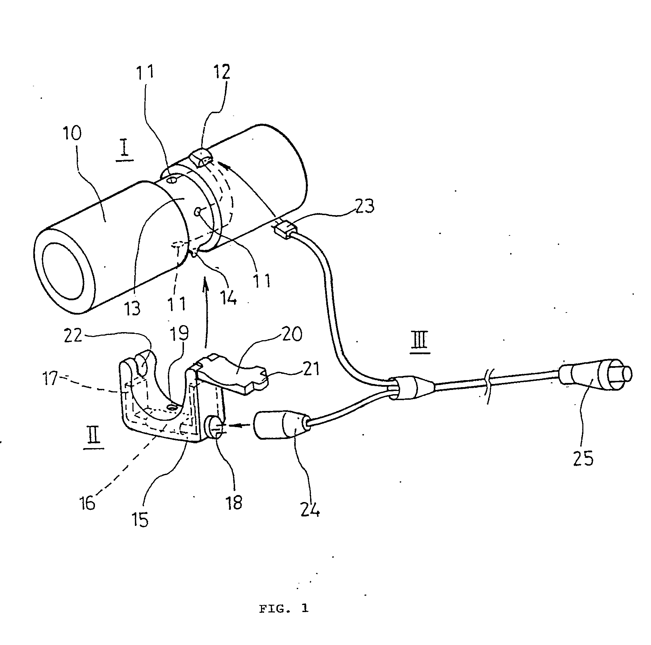

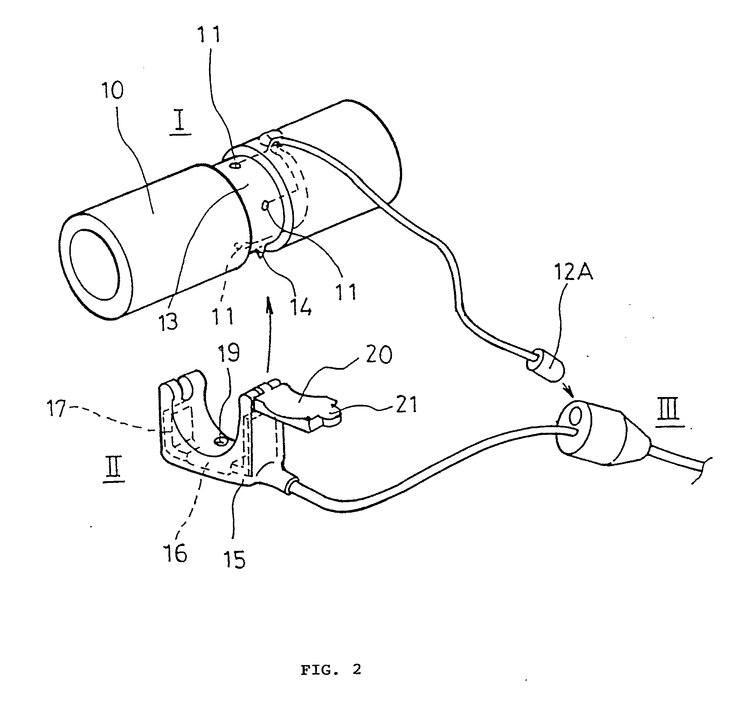

Fig. 1 is a view showing the construction of one preferred embodiment of the present invention; Fig. 2 is a view showing the construction of another embodiment of the invention; Fig. 3 is a view showing the construction of a conventional electromagnetic blood flowmeter probe; and Fig. 4 is a view explanatory of the principle of an electromagnetic blood flowmeter.

## DETAILED DESCRIPTION OF THE PRESENT INVENTION

Figs. 1 is a view showing the construction of one preferred embodiment of an electromagnetic blood flowmeter probe of the present invention. This probe comprises an electrode portion I, an exciter coil portion II detachably connectable to this electrode portion, and a connecting cord portion III.

The electrode portion I includes a hollow bobbin 10 through which the blood flows, electrodes 11 mounted on the bobbin 10, and an electrode connector 12 connected to the electrodes 11. The bobbin 10 has a guide 13 and a projection 14 which serve as positioning means when the exciter coil portion II is attached to the bobbin. The exciter coil portion II comprises a U-shaped coil portion body 15 fixedly attachable to the bobbin 10 of the electrode portion I, an exciter coil 16 for applying a magnetic field to the blood flow passing through the interior of the bobbin 10, an iron core 17, and an exciter coil connector 18 connected to the exciter coil 16. The exciter coil 16 and the iron core 17 are embedded in the body 15, and the body 15 has a recess 19 for engagement with the projection 14 of the bobbin 10. A fixing plate 20 is hingedly mounted on the body 15 so as to close an open end of the U-shaped body 15. A retaining piece 21 is projectingly formed on the fixing plate 20, and is adapted to fit in a fixing groove 22 formed in the body 15. The connecting cord portion III includes an electrode connector 23 connectable to the connector 12 of the electrode portion I, an exciter coil connector 24 connectable to the connector 18 of the exciter coil portion II, and a connector 25 connectable to a blood flowmeter (not shown).

In the above electromagnetic blood flowmeter probe of the separating type, the hollow bobbin 10 is first connected to a blood circuit. At this time, the electrodes 11 and the electrode connector 12 have been mechanically fixed by the bobbin 10, and have been electrically insulated by the bobbin. Then, the coil portion body 15 is attached to the bobbin 10 as indicated by an arrow. At this time, since the guide 13, the projection 14 and the recess 19, which serve as a positioning mechanism for positioning the bobbin 10 and the coil portion body 15 relative to each other when they are connected together, are provided on them, they can be easily positioned and connected together by attaching the coil portion body 15 to the bobbin 10 along the guide 13 and by fitting the projection 14 of the bobbin 10 in the recess 19. Also, at this time, the mechanical relative position between the electrodes 11 and the exciter coil 16 can be fixed. Then, the fixing plate 20 is turned so as to fit the retaining piece 21 in the fixing groove 22. By doing so, the electrode portion I and the exciter coil portion II are integrally connected together. Thereafter, the connectors 23 to 25 of the connecting cord portion III are connected to the connectors 12 and 18 and the blood flowmeter, respectively, thereby completing preparations for the blood flow measurement.

The operation for the above blood flow measurement is carried out in the same manner as in the prior art. When the exciter coil 16 applies the magnetic field to the blood flow passing through the interior of the bobbin 10, an electromotive force proportional to the velocity of this blood flow is produced. This electromotive force is taken out by the electrodes 11, and is inputted via the connecting cord portion III into the blood flowmeter where it is amplified, and then it is recorded and displayed as a detection value of the blood flow.

As described above, the electrode portion I and the exciter coil portion II are separate from each other, and the mechanical relative position between them when they are connected together can be kept constant. Therefore, the cost can be reduced while maintaining the precision heretofore achieved. Namely, only the inexpensive electrode portion I, as well as the blood circuit parts, is disposable, and the exciter coil portion II can be reused. Further, the electrode portion I incorporated in the blood circuit can be subjected to a sterilizing treatment, and therefore the incorporation is not cumbersome.

Fig. 2 shows the construction of another embodiment of this invention. In this embodiment, a connector 12A of an electrode portion I is mounted on a distal end of a cord led from a bobbin 10, and an exciter coil portion II has no connector, and is adapted to be connected directly to the blood flowmeter through a cord.

Even with this construction, similar effects as in the above embodiment can be obtained.

As described above, in the present invention, the electrode portion and the exciter coil portion are separate from each other, and the positioning mechanism for fixing the mechanical relative position between the electrode portion and the exciter coil portion when they are connected together is provided on the bobbin of the electrode portion and the body of the exciter coil portion. Therefore, the electrode portion, as well as the blood circuit parts, is disposable, and the incorporation is not cumbersome.

**Claims**

1. An electromagnetic blood flowmeter probe comprising:

an electrode portion having electrodes provided on a hollow bobbin through which a blood flow passes;

an exciter coil portion having a body in which an exciter coil for applying a magnetic field to said blood flow is embedded; and

a positioning means for fixing a relative position between said electrode portion and said exciter coil portion when they are connected together is provided on said bobbin of said electrode portion and said body of said exciter coil portion.

2. An electromagnetic blood flowmeter probe as calimed in claim 1, further comprising:

connecting cord portion including: and

a first electrode connector connectable to a connector of said electrode portion; and

a second electrode connector connectable to a connector of said exciter coil portion.

3. An electromagnetic blood flowmeter probe as claimed in claim 1, further comprising:

connecting cord portion including a pair of cable for directly connecting said probe and coil portions to said connecting cord portion, respectively.

4. An electromagnetic blood flowmeter probe as claimed in claim 1, wherein said position means includes a guide and a projection formed on said body of said coil portion, and a recess disposed on said bobbin.

FIG. 1

FIG. 2

FIG. 3

FIG. 4

European
Patent Office

EUROPEAN SEARCH
REPORT

Application Number

EP 91 10 8858

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 881 413   (D.K. GEORGI et al.)<br>* column 4, line 54 - column 10, line 43; figures 1-13 * | 1,3 | A 61 B 5/0265 |
| A | | 2,4 | |
| | – – – | | |
| A | US-A-4 346 604   (R.K. SNOOK et al.)<br>* column 2, lines 33-51; column 4, line 15 - column 5, line 31; column 7, line 47 - column 8, line 21; figures 1-3 * | 1,2,4 | |
| | – – – | | |
| A | US-A-4 118 981   (E.K. CAVE)<br>* column 2, line 60 - column 4, line 11; figures 1-4 * | 1,2 | |
| | – – – | | |
| A | US-A-3 592 187   (M. YOUDIN)<br>* column 2, line 1 - column 3, line 33; figures 1,2 * | 1 | |
| | – – – – – | | |

TECHNICAL FIELDS
SEARCHED (Int. Cl.5)

A 61 B 5/00
G 01 F 1/00

The present search report has been drawn up for all claims

| Place of search | Date of completion of search | Examiner |
|---|---|---|
| Berlin | 21 August 91 | WEIHS J.A. |